# EUROPÄISCHE PATENTSCHRIFT

(11) **EP 1 020 455 B1**
(45) Veröffentlichungstag und Bekanntmachung des Hinweises auf die Patenterteilung: **05.03.2003**
(21) Anmeldenummer: 99125083.8
(22) Anmeldetag: 16.12.1999
(51) Int. Cl.: C07D 295/088

(54) **Verfahren zur Herstellung von 2,2'-Dimorpholinodiethylether**
Process for the preparation of 2,2'-dimorpholinodiethylether
Procédé de préparation de 2,2'-dimorpholinodiéthyléther

(30) Priorität: 14.01.1999 DE 19901198
(43) Veröffentlichungstag der Anmeldung: 19.07.2000
(73) Patentinhaber: BASF AKTIENGESELLSCHAFT, 67056 Ludwigshafen (DE)
(72) Erfinder: Riechers, Hartmut, Dr., 67435 Neustadt (DE); Simon, Joachim, Dr., 68161 Mannheim (DE); Henne, Andreas, Dr., 67433 Neustadt (DE); Höhn, Arthur, Dr., 67281 Kirchheim (DE)

(56) Entgegenhaltungen:
- EP-A- 0 036 331
- EP-A- 0 070 397
- US-A- 3 155 657
- US-A- 3 645 925
- US-A- 3 817 997

## Beschreibung

Die vorliegende Erfindung betrifft ein Verfahren zur Herstellung von 2,2'-Dimorpholinodiethylether (DMDEE) der Formel I 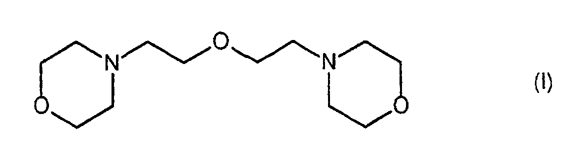 durch Umsetzung von Diethylenglykol (DEG) der Formel II 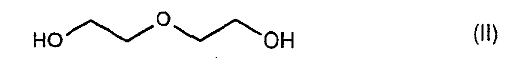 mit Ammoniak unter Druck und bei erhöhter Temperatur in Gegenwart von Wasserstoff und einem Hydrierkatalysator.

2,2'-Dimorpholinodiethylether (I) ist ein bekannter Polymerisationskatalysator bei der Herstellung von Polyurethanen (vgl. z. B.: US-A-3,645,925).

HU-A-212713 (Derwent Abstract Nr. 97-539345/50) beschreibt die Herstellung von 2,2'-Dimorpholinodiethylether durch katalytische reduktive Aminierung von Diethylenglykol mit Morpholin in Gegenwart eines Kupfer-, Chrom- und Barium-haltigen Katalysators.

EP-A-716 084 betrifft ein Herstellverfahren für 2,2'-Dimorpholinodiethylether und N-(2(2-Hydroxyethoxy)ethyl)morpholin durch Umsetzung von Diethylenglykol mit Morpholin in Gegenwart von H₂ und einem Kupferkatalysator und in Abwesenheit von Ammoniak (vgl. loc. cit.: Seite 3, Zeilen 53 bis 54).

JP-A-02 111 765 (Derwent Abstract Nr. 90-169102/22) beschreibt die Herstellung von 2,2'-Dimorpholinodiethylethern durch Umsetzung von gegebenenfalls substituierten Morpholinen mit Diethylenglykol in Gegenwart eines Raney-Cobalt-Katalysators in Ausbeuten von 67 bis 94 %.

EP-A-36 331 und US-A-4,647,663 beschreiben ein Verfahren zur Herstellung von Morpholin und Morpholinderivaten, bei gleichzeitig geringem Anfall von 2,2'-Dimorpholinodiethylether als unerwünschtem Nebenprodukt (vgl. EP-A-36 331, Seite 4, Zeilen 19 bis 23), durch Umsetzung eines Dialkylenglykols mit Ammoniak in Gegenwart von H₂ und einem Hydrierkatalysator in einem Rieselbettreaktor.

US-A-3,817,997 beschreibt ein Verfahren zur Herstellung von 2,2'-Dimorpholinodiethylether durch Behandlung eines Amin-Rückstandes, der bei einer katalytischen Reaktion von Diethylenglykol mit Ammoniak anfällt, mit einem Hydrierkatalysator bei erhöhter Temperatur und erhöhtem Druck.

Chem. Prum. (1992), 42(5-6), 105-9 (Chem. Abstr. 119: 72556) beschreibt eine gaschromatographische Analyse von Nebenprodukten in Rohausträgen der Morpholin-Synthese aus Diethylenglykol und NH₃. Danach fällt 2,2'-Dimorpholinodiethylether als Nebenprodukt in größeren Mengen an, wenn die Morpholin-Synthese statt in der Flüssigphase in der Gasphase durchgeführt wird.

Adv. Chem. Ser. 1992, 230 (Homogeneous Transition Met. Catal. React.), 433-42 berichtet über die Bildung von 2,2'-Dimorpholinodiethylether mit einer Selektivität und Ausbeute von nur 17 % bei der Reaktion von Diethylenglykol mit Morpholin in Gegenwart eines RuCl₃ • xH₂O * 3 PBu₃- Katalysators (loc. cit.: Tabelle V auf Seite 440). Nachteilig ist hier die Verwendung eines teuren Edelmetallkatalysators, der sich zudem nur aufwendig zurückführen lässt.

Khim. Prom-st. (Moscow) (11), 653-5 (1982) (Chem. Abstr. 98: 91383q) beschreibt die Herstellung von Morpholin durch Gasphasen-Cycloaminierung von Diethylenglykol mit Ammoniak in Gegenwart von H₂ und einem Ni-Cu-Cr₂O₃-Katalysator.

Zh. Vses. Khim. Obshchest. 14(5), 589-90 (1969) (Chem. Abstr. 72: 66879m) beschreibt die Entstehung von Morpholin in 70 % Ausbeute durch Gasphasenreaktion von Diethylenglykol mit NH₃ an einem Nikkel-Katalysator in Gegenwart von H₂. Als Nebenprodukt wurde dabei u.a. auch 2,2'-Dimorpholinodiethylether in 5 bis 7 % Ausbeute isoliert.

Die vorstehend beschriebenen Verfahren liefern 2,2'-Dimorpholinodiethylether entweder nur in sehr kleiner Ausbeute oder gehen von teuren Ausgangsstoffen aus.

Der vorliegenden Erfindung lag daher die Aufgabe zugrunde, ein alternatives wirtschaftliches Verfahren zur Herstellung von 2,2'-Dimorpholinodiethylether aufzufinden.

Demgemäß wurde ein Verfahren zur Herstellung von 2,2'-Dimorpholinodiethylether durch Umsetzung von Diethylenglykol mit Ammoniak unter Druck und bei erhöhter Temperatur in Gegenwart von Wasserstoff und einem Hydrierkatalysator gefunden, welches dadurch gekennzeichnet ist, dass die katalytisch aktive Masse des Katalysators vor dessen Reduktion mit Wasserstoff

| | |
|---|---|
| 20 bis 85 Gew.-% | Aluminiumoxid (Al₂O₃), Zirkoniumdioxid (ZrO₂), Titandioxid (TiO₂) und/oder Siliziumdioxid (SiO₂), |
| | |
| 1 bis 70 Gew.-% | sauerstoffhaltige Verbindungen des Kupfers, berechnet als CuO, |
| | |
| 0 bis 50 Gew.-% | sauerstoffhaltige Verbindungen des Magnesiums, berechnet als MgO, sauerstoffhaltige Verbindungen des Chroms, berechnet als Cr₂O₃, sauerstoffhaltige Verbindungen des Zinks, berechnet als ZnO, sauerstoffhaltige Verbindungen des Bariums, berechnet als BaO, und/oder sauerstoffhaltige Verbindungen des Calciums, berechnet als CaO, und |
| | |
| weniger als 10 Gew.-% | sauerstoffhaltige Verbindungen des Nikkels, berechnet als NiO, bezogen auf die sauerstoffhaltigen Verbindungen des Kupfers, berechnet als CuO, |

enthält.

Im erfindungsgemäßen Verfahren werden die Katalysatoren bevorzugt in Form von Katalysatoren eingesetzt, die nur aus katalytisch aktiver Masse und gegebenenfalls einem Verformungshilfsmittel (wie z. B. Graphit oder Stearinsäure), falls der Katalysator als Formkörper eingesetzt wird, bestehen, also keine weiteren katalytisch aktiven Begleitstoffe enthalten.

In diesem Zusammenhang werden die oxidischen Trägermaterialien Titandioxid (TiO₂), Aluminiumoxid (Al₂O₃), Zirkoniumdioxid (ZrO₂) und Siliziumdioxid (SiO₂) als zur katalytisch aktiven Masse gehörig gewertet.

Die Katalysatoren werden dergestalt eingesetzt, dass man die katalytisch aktive, zu Pulver vermahlene Masse in das Reaktionsgefäss einbringt oder, dass man die katalytisch aktive Masse nach Mahlung, Vermischung mit Formhilfsmitteln, Formung und Temperung als Katalysatorformkörper - beispielsweise als Tabletten, Kugeln, Ringe, Extrudate (z. B. Stränge) - im Reaktor anordnet.

Die Konzentrationsangaben (in Gew.-%) der Komponenten des Katalysators beziehen sich jeweils - falls nicht anders angegeben - auf die katalytisch aktive Masse des fertigen Katalysators nach dessen letzter Wärmebehandlung und vor dessen Reduktion mit Wasserstoff.

Die katalytisch aktive Masse des Katalysators, nach dessen letzter Wärmebehandlung und vor dessen Reduktion mit Wasserstoff, ist als die Summe der Massen der katalytisch aktiven Bestandteile und der o. g. Katalysatorträgermaterialien definiert und enthält im wesentlichen die folgenden Bestandteile:
- Titandioxid (TiO₂) und/oder Aluminiumoxid (Al₂O₃) und/oder Zirkoniumdioxid (ZrO₂) und/oder Siliziumdioxid (SiO₂)
- und sauerstoffhaltige Verbindungen des Kupfers
- und optional sauerstoffhaltige Verbindungen des Magnesiums und/oder des Chroms und/oder des Zinks und/oder des Bariums und/oder des Calciums
- und optional sauerstoffhaltige Verbindungen des Nickels, wobei die Menge dieser sauerstoffhaltigen Verbindungen des Nikkels, berechnet als NiO, bezogen auf die Menge an sauerstoffhaltigen Verbindungen des Kupfers, berechnet als CuO, weniger als 10 Gew.-% beträgt.

Die Summe der o. g. Bestandteile der katalytisch aktiven Masse, berechnet als Al₂O₃, ZrO₂, TiO₂, SiO₂, CuO, MgO, Cr₂O₃, ZnO, BaO, CaO und NiO beträgt üblicherweise 70 bis 100 Gew.-%, bevorzugt 80 bis 100 Gew.-%, besonders bevorzugt 90 bis 100 Gew.-%, ganz besonders bevorzugt 100 Gew.-%.

Die katalytisch aktive Masse der im erfindungsgemäßen Verfahren eingesetzten Katalysatoren kann weiterhin ein oder mehrere Elemente (Oxidationsstufe 0) oder deren anorganische oder organische Verbindungen, ausgewählt aus den Gruppen I A bis VI A und I B bis VII B und VIII des Periodensystems, enthalten.

Beispiele für solche Elemente bzw. deren Verbindungen sind:

Übergangsmetalle, wie Co bzw. CoO, Re bzw. Rheniumoxide, Mn bzw. MnO₂, Mo bzw. Molybdänoxide, W bzw. Wolframoxide, Ta bzw. Tantaloxide, Nb bzw. Nioboxide oder Nioboxalat, V bzw. Vanadiumoxide bzw. Vanadylpyrophosphat; Lanthanide, wie Ce bzw. CeO₂ oder Pr bzw. Pr₂O₃; Alkalimetalloxide, wie Na₂O; Alkalimetallcarbonate; Erdalkalimetalloxide, wie SrO; Erdalkalimetallcarbonate, wie MgCO₃, CaCO₃ und BaCO₃; Boroxid (B₂O₃).

Die katalytisch aktive Masse der im erfindungsgemäßen Verfahren eingesetzten Katalysatoren enthält nach deren letzter Wärmebehandlung und vor dessen Reduktion mit Wasserstoff
- 20 bis 85 Gew.-%, bevorzugt 25 bis 80 Gew.-%, besonders bevorzugt 30 bis 75 Gew.-%, Aluminiumoxid (Al₂O₃) und/oder Zirkoniumdioxid (ZrO₂) und/oder Titandioxid (TiO₂) und/oder Siliziumdioxid (SiO₂) und
- 1 bis 70 Gew.-%, bevorzugt 2 bis 65 Gew.-%, besonders bevorzugt 5 bis 60 Gew.-%, ganz besonders bevorzugt 20 bis 60 Gew.-%, sauerstoffhaltige Verbindungen des Kupfers, berechnet als CuO,
- 0 bis 50 Gew.-%, bevorzugt 0 bis 30 Gew.-%, besonders bevorzugt 0 bis 20 Gew.-%, sauerstoffhaltige Verbindungen des Magnesiums, berechnet als MgO, und/oder sauerstoffhaltige Verbindungen des Chroms, berechnet als Cr₂O₃, und/oder sauerstoffhaltige Verbindungen des Zinks, berechnet als ZnO, und/oder sauerstoffhaltige Verbindungen des Bariums, berechnet als BaO, und/oder sauerstoffhaltige Verbindungen des Calciums, berechnet als CaO, und
- weniger als 10 Gew.-%, bevorzugt weniger als 5 Gew.-%, ganz besonders bevorzugt weniger als 1 Gew.-%, beispielsweise 0 Gew.-%, sauerstoffhaltige Verbindungen des Nickels, berechnet als NiO, bezogen auf die sauerstoffhaltigen Verbindungen des Kupfers, berechnet als CuO.

Bevorzugte Katalysatoren enthalten in ihrer katalytischen Masse 20 bis 85 Gew.-%, bevorzugt 25 bis 80 Gew.-%, besonders bevorzugt 30 bis 75 Gew.-%, Aluminiumoxid (Al₂O₃) und/oder Siliziumdioxid (SiO₂) und keine sauerstoffhaltigen Verbindungen des Zirkoniums und Titans.

Bei den sauerstoffhaltigen Verbindungen des Kupfers handelt es sich insbesondere um Kupfer-(I)-oxid und Kupfer-(II)-oxid, bevorzugt um Kupfer-(II)-oxid.

Zur Herstellung der im erfindungsgemäßen Verfahren verwendeten Katalysatoren sind verschiedene Verfahren möglich. Sie sind beispielsweise durch Peptisieren pulvriger Mischungen der Hydroxide, Carbonate, Oxide und/oder anderer Salze der Komponenten Aluminium, Zirkonium, Titan, Silizium, Kupfer, Magnesium, Chrom, Zink, Barium und Calcium mit Wasser und nachfolgendes Extrudieren und Tempern der so erhaltenen Masse erhältlich.

Die im erfindungsgemäßen Verfahren verwendeten Katalysatoren können auch durch Tränkung von Zirkoniumdioxid (ZrO₂), Titandioxid (TiO₂), Aluminiumoxid (Al₂O₃), Siliziumdioxid (SiO₂) oder Gemischen zweier oder mehrerer dieser anorganischen Oxide, die beispielsweise in Form von Pulver oder Formkörpern, wie Strängen, Tabletten, Kugeln oder Ringen, vorliegen, hergestellt werden.

Aluminiumoxid kann dabei in verschiedenen Modifikationen eingesetzt werden, bevorzugt sind α-, γ- oder θ-Al₂O₃.

Zirkoniumdioxid wird beispielsweise in der monoklinen oder tetragonalen Form, bevorzugt in der monoklinen Form, und Titandioxid vorzugsweise als Anatas oder Rutil eingesetzt.

Als Trägermaterial geeignetes Siliziumdioxid kann z. B. über eine Fällung aus Wasserglas oder über das Sol-Gel-Verfahren erhalten werden oder als mesoporöses SiO₂ oder Kieselgel (z. B. nach Ullmann, Enzykl. Techn. Chem., 4. Auflage, Band 21, S. 457-63, 1982) oder in Form von Silikaten, wie Bentonit, Montmorillonit, Kaolin, Hectorit oder Alumosilikaten (z. B. gemäß Nature, Band 359, S. 710-12, 1992 oder Alkali- bzw. Erdalkali-Alumosilikaten (Zeolithe), z. B. der allgemeinen Formel M_{2/z}O • Al₂O₃ • xSiO₂ • yH₂O, wobei M ein ein- oder mehrwertiges Metall, H, [NH₄], z die Wertigkeit, x = 1,8 bis ca. 12 und y = 0 bis ca. 8 bedeuten), Magnesiumsilikaten (z. B. Steatit), Zirkoniumsilikaten, Cersilikaten oder Calciumsilikaten eingesetzt werden.

Die Herstellung von Formkörpern der o.g. anorganischen Oxide kann nach den üblichen Verfahren erfolgen.

Die Tränkung dieser anorganischen Oxide erfolgt ebenfalls nach den üblichen Verfahren, wie z. B. in EP-A-599 180, EP-A-673 918 oder A. B. Stiles, Catalyst Manufacture -Laboratory and Commercial Preparations, Marcel Dekker, New York (1983) beschrieben, durch Aufbringung einer jeweils entsprechenden Metallsalzlösung in einer oder mehreren Tränkstufen, wobei als Metallsalze z. B. entsprechende Nitrate, Acetate oder Chloride verwendet werden.

Die Masse wird im Anschluss an die Tränkung getrocknet und ggf. kalziniert.

Die Tränkung kann nach der sogenannten "incipient wetness"-Methode erfolgen, bei der das anorganische Oxid, bzw. das Gemisch der anorganischen Oxide, entsprechend seiner Wasseraufnahmekapazität maximal bis zur Sättigung mit der Tränklösung befeuchtet wird. Die Tränkung kann aber auch in überstehender Lösung erfolgen.

Bei mehrstufigen Tränkverfahren ist es zweckmäßig, zwischen einzelnen Tränkschritten zu trocknen und ggf. zu kalzinieren. Die mehrstufige Tränkung ist vorteilhaft besonders dann anzuwenden, wenn das anorganische Oxid, bzw. das Gemisch der anorganischen Oxide, mit einer größeren Metallmenge beaufschlagt werden soll.

Zur Aufbringung mehrerer Metallkomponenten auf das anorganische Oxid, bzw. das Gemisch der anorganischen Oxide, kann die Tränkung gleichzeitig mit allen Metallsalzen oder in beliebiger Reihenfolge der einzelnen Metallsalze nacheinander erfolgen.

Im allgemeinen werden zur Herstellung der im erfindungsgemäßen Verfahren verwendeten Katalysatoren jedoch Fällungsmethoden angewandt. So können sie beispielsweise durch eine gemeinsame Fällung der Kupfer-, Magnesium-, Chrom-, Zink-, Barium und Calcium-Kompnenten aus einer diese Elemente enthaltenden, wässrigen Salzlösung mittels Mineralbasen in Gegenwart einer Aufschlämmung einer schwerlöslichen, sauerstoffhaltigen Aluminium-, Titan-, Siliziumund/oder Zirkonium-Verbindung und anschließendes Waschen, Trocknen und Calcinieren des erhaltenen Präzipitats erhalten werden. Als schwerlösliche, sauerstoffhaltige Aluminium-, Titan-, Silizium- und/oder Zirkonium-Verbindungen können beispielsweise Aluminiumoxid, Titandioxid, Siliziumdioxid, Zirkoniumdioxid und Zirkoniumoxidhydrat Verwendung finden. Die Aufschlämmungen der schwerlöslichen Aluminium-, Titan-, Silizium- und/oder Zirkonium-Verbindungen können durch Suspendieren feinkörniger Pulver dieser Verbindungen in Wasser unter kräftigem Rühren hergestellt werden. Vorteilhaft werden diese Aufschlämmungen durch Ausfällen der schwerlöslichen Aluminium-, Titan-, Silizium- und/oder Zirkonium-Verbindungen aus wässrigen Aluminium-, Titan-, Silizium- und/oder Zirkonium-Salzlösungen mittels Mineralbasen erhalten.

Bevorzugt werden die im erfindungsgemäßen Verfahren verwendeten Katalysatoren über eine gemeinsame Fällung (Mischfällung) aller ihrer Komponenten hergestellt. Dazu wird zweckmäßigerweise eine die Katalysatorkomponenten enthaltende, wässrige Salzlösung in der Wärme und unter Rühren so lange mit einer wässrigen Mineralbase, insbesondere einer Alkalimetallbase - beispielsweise Natriumcarbonat, Natriumhydroxid, Kaliumcarbonat oder Kaliumhydroxid - versetzt, bis die Fällung vollständig ist. Die Art der verwendeten Salze ist im allgemeinen nicht kritisch: Da es bei dieser Vorgehensweise vornehmlich auf die Wasserlöslichkeit der Salze ankommt, ist ein Kriterium ihre, zur Herstellung dieser verhältnismäßig stark konzentrierten Salzlösungen erforderliche, gute Wasserlöslichkeit. Es wird als selbstverständlich erachtet, daß bei der Auswahl der Salze der einzelnen Komponenten natürlich nur Salze mit solchen Anionen gewählt werden, die nicht zu Störungen führen, sei es, indem sie unerwünschte Fällungen verursachen oder indem sie durch Komplexbildung die Fällung erschweren oder verhindern.

Die bei diesen Fällungsreaktionen erhaltenen Niederschläge sind im allgemeinen chemisch uneinheitlich und bestehen u.a. aus Mischungen der Oxide, Oxidhydrate, Hydroxide, Carbonate und unlöslichen und basischen Salze der eingesetzten Metalle. Es kann sich für die Filtrierbarkeit der Niederschläge als günstig erweisen, wenn sie gealtert werden, d.h. wenn man sie noch einige Zeit nach der Fällung, gegebenenfalls in Wärme oder unter Durchleiten von Luft, sich selbst überläßt.

Die nach diesen Fällungsverfahren erhaltenen Niederschläge werden wie üblich zu den erfindungsgemäßen Katalysatoren weiterverarbeitet. Nach dem Waschen werden sie im allgemeinen bei 80 bis 200 °C, vorzugsweise bei 100 bis 150 °C, getrocknet und danach calciniert. Die Calcinierung wird im allgemeinen bei Temperaturen zwischen 300 und 800 °C, vorzugsweise 400 bis 600 °C, insbesondere 450 bis 550 °C, ausgeführt.

Nach der Calcinierung wird der Katalysator zweckmäßigerweise konditioniert, sei es, daß man ihn durch Vermahlen auf eine bestimmte Korngröße einstellt oder daß man ihn nach seiner Vermahlung mit Formhilfsmitteln wie Graphit oder Stearinsäure vermischt, mittels einer Presse zu Formlingen, z. B. Tabletten, verpreßt und tempert. Die Tempertemperaturen entsprechen dabei im allgemeinen den Temperaturen bei der Calcinierung.

Die auf diese Weise hergestellten Katalysatoren enthalten die katalytisch aktiven Metalle in Form eines Gemisches ihrer sauerstoffhaltigen Verbindungen, d.h. insbesondere als Oxide und Mischoxide.

Die auf diese Weise hergestellten Katalysatoren werden als solche gelagert und ggf. gehandelt. Vor ihrem Einsatz als Katalysatoren zur Herstellung von 2,2'-Dimorpholinodiethylether aus Diethylenglykol und Ammoniak werden sie üblicherweise vorreduziert. Sie können jedoch auch ohne Vorreduktion eingesetzt werden, wobei sie dann unter den Bedingungen der hydrierenden Aminierung durch den im Reaktor vorhandenen Wasserstoff reduziert werden.

Zur Vorreduktion werden die Katalysatoren im allgemeinen zunächst bei 150 bis 200 °C über einen Zeitraum von 12 bis 20 Stunden einer Stickstoff-Wasserstoff-Atmosphäre ausgesetzt und anschließend noch bis zu ca. 24 Stunden bei 200 bis 400 °C in einer Wasserstoffatmosphäre behandelt. Bei dieser Vorreduktion wird ein Teil der in den Katalysatoren vorliegenden sauerstoffhaltigen Metallverbindungen zu den entsprechenden Metallen reduziert, so daß diese gemeinsam mit den verschiedenartigen Sauerstoffverbindungen in der aktiven Form des Katalysators vorliegen.

Die in EP-A-284 919 offenbarten Katalysatoren der allgemeinen Formel MₓMg_{y}(SiO₂)•nH₂O, worin M ein zweiwertiges, reduzierbares Metallatom aus der Gruppe Cu, Fe, Co und Ni, x und y Zahlen sind, die zusammen den Wert 1,5 erreichen können, und n nach Trocknung ausgedrückt in Gew.-% zwischen 0 und 80 liegt, beispielsweise der in loc. cit im Beispiel beschriebene Katalysator enthaltend 35 % CuO, 9 % MgO und 38 % SiO₂ und der in EP-A-863 140 auf Seite 3 beschriebene Katalysator enthaltend 45 bis 47 Gew.-% CuO, Magnesiumsilikat aus etwa 15 bis 17 Gew.-% MgO und 35 bis 36 Gew.-% SiO₂, etwa 0,9 Gew.-% Cr₂O₃, etwa 1 Gew.-% BaO und etwa 0,6 Gew.-% ZnO, und
die in WO 95/32171 und EP-A-816 350 offenbarten Trägerkatalysatoren enthaltend 5 bis 50, bevorzugt 15 bis 40, Gew.-% Kupfer, berechnet als CuO, 50 bis 95, bevorzugt 60 bis 85, Gew.-% Silicium, berechnet als SiO₂, 0 bis 20 Gew.-% Magnesium, berechnet als MgO, 0 bis 5 Gew.-% Barium, berechnet als BaO, 0 bis 5 Gew.-% Zink, berechnet als ZnO, und 0 bis 5 Gew.-% Chrom, berechnet als Cr₂O₃, jeweils bezogen auf das Gesamtgewicht des calcinierten Katalysators, beispielsweise der in EP-A-816 350, Seite 5, offenbarte Katalysator enthaltend 30 Gew.-% CuO und 70 Gew.-% SiO₂,
sind im erfindungsgemäßen Verfahren bevorzugt einsetzbar.

Besonders bevorzugt sind im erfindungsgemäßen Verfahren die in DE-A-24 45 303 offenbarten Katalysatoren, die durch Temperung eines basischen Kupfer und Aluminium enthaltenen Carbonats der allgemeinen Zusammensetzung CuₘAl₆(CO₃)_{0,5m}O₃(OH)ₘ₊₁₂, wobei m einen beliebigen, auch nicht ganzzahligen, Wert zwischen 2 und 6 bedeutet, bei einer Temperatur von 350 bis 700 °C erhältlich sind, beispielsweise der in loc. cit., Beispiel 1, offenbarte kupferhaltige Fällkatalysator, der durch Behandlung einer Lösung von Kupfernitrat und Aluminiumnitrat mit Natriumbicarbonat und anschließendem Waschen, Trocknen und Tempern des Präzipitats hergestellt wird, einsetzbar.

Das erfindungsgemäße Verfahren läßt sich diskontinuierlich oder bevorzugt kontinuierlich wie folgt durchführen, wobei der Katalysator bevorzugt als Festbett im Reaktor angeordnet ist.

Die Aminierung des Diethylenglykols kann in der Flüssigphase oder in der Gasphase durchgeführt werden. Bevorzugt ist das Festbettverfahren in der Gasphase.

Ammoniak wird im allgemeinen im Molverhältnis von Ammoniak zu Diethylenglykol von 1 : 1 bis 50 : 1, bevorzugt 1,5 : 1 bis 30 : 1, besonders bevorzugt 2 : 1 bis 20 : 1, ganz besonders bevorzugt 2 : 1 bis 6 : 1, eingesetzt. Der Ammoniaküberschuß kann auch größer als 50 : 1 sein.

Der Wasserstoff wird der Reaktion im allgemeinen in einer Menge von 5 bis 400 l, bevorzugt in einer Menge von 50 bis 200 l pro Mol Alkoholkomponente zugeführt, wobei die Literangaben jeweils auf Normalbedingungen umgerechnet wurden (S.T.P.).

Beim Arbeiten in der Flüssigphase leitet man die Edukte Diethylenglykol und Ammoniak in flüssiger Phase bei Drücken von 0,1 bis 30 MPa, bevorzugt 10 bis 25 MPa, besonders bevorzugt 15 bis 20 MPa, und Temperaturen von 100 bis 300 °C, bevorzugt 150 bis 250 °C, besonders bevorzugt 170 bis 230 °C, inklusive Wasserstoff über den Katalysator, der sich üblicherweise in einem bevorzugt von außen beheizten Festbettreaktor (z.B. Rohrreaktor) befindet. Es ist dabei sowohl eine Rieselfahrweise als auch eine Sumpffahrweise möglich. Die Katalysatorbelastung liegt im allgemeinen im Bereich von 0,05 bis 1,0 kg DEG pro Liter Katalysator (Schüttvolumen) und Stunde. Gegebenenfalls kann eine Verdünnung der Edukte mit einem geeigneten Lösungsmittel, wie N-Methylpyrrolidon, erfolgen. Es ist zweckmäßig, die Reaktanden bereits vor der Zuführung in das Reaktionsgefäß zu erwärmen, und zwar bevorzugt auf die Reaktionstemperatur.

Beim Arbeiten in der Gasphase wird Diethylenglykol in einem zur Verdampfung ausreichend groß gewählten Gasstrom enthaltend Wasserstoff und Ammoniak, bevorzugt bestehend aus Wasserstoff und Ammoniak, bei Drücken von 0,1 bis 10 MPa, bevorzugt 1 bis 5 MPa, besonders bevorzugt 1 bis 3 MPa, und Temperaturen von 100 bis 300 °C, bevorzugt 150 bis 250 °C, besonders bevorzugt 170 bis 230°C, über den Katalysator geleitet. Es ist dabei sowohl eine Anströmung des Katalysatorfestbetts von oben als auch von unten möglich. Den erforderlichen Gasstrom erhält man bevorzugt durch eine Kreisgasfahrweise, wobei beispielsweise bei einem Katalysatorschüttvolumen von 1,5 l eine Kreisgasmenge von ca. 9 bis 13 m³/h (Volumen auf Normalbedingungen umgerechnet) und eine Abgasmenge von ca. 250 bis 350 l/h gefahren wird. Die Katalysatorbelastung liegt im allgemeinen im Bereich von 0,1 bis 0,8, bevorzugt 0,2 bis 0,4, kg DEG pro Liter Katalysator (Schüttvolumen) und Stunde.

Sowohl beim Arbeiten in der Flüssigphase als auch beim Arbeiten in der Gasphase ist die Anwendung höherer Temperaturen, höherer Gesamtdrücke und höherer Katalysatorbelastungen möglich. Der Druck im Reaktionsgefäß, welcher sich aus der Summe der Partialdrücke des Aminierungsmittels, der Alkoholkomponente und der gebildeten Reaktionsprodukte sowie ggf. des mitverwendeten Lösungsmittels bei den angegebenen Temperaturen ergibt, wird zweckmäßigerweise durch Aufpressen von Wasserstoff auf den gewünschten Reaktionsdruck erhöht.

Sowohl beim kontinuierlichen Arbeiten in der Flüssigphase als auch beim kontinuierlichen Arbeiten in der Gasphase kann der überschüssige Ammoniak zusammen mit dem Wasserstoff im Kreis geführt werden.

Das im Zuge der Umsetzung gebildete Reaktionswasser wirkt sich im allgemeinen auf den Umsetzungsgrad, die Reaktionsgeschwindigkeit, die Selektivität und die Katalysatorstandzeit nicht störend aus und wird deshalb zweckmäßigerweise erst bei der Aufarbeitung des Reaktionsproduktes aus diesem entfernt, z. B. destillativ.

Aus dem Reaktionsaustrag werden, nachdem dieser zweckmäßigerweise entspannt worden ist, der überschüssige Ammoniak und der Wasserstoff entfernt und das erhaltene Reaktionsrohprodukt, das im wesentlichen 2,2'-Dimorpholinodiethylether, Morpholin, Wasser und evtl. unumgesetztes Diethylenglykol enthält, durch eine fraktionierende Rektifikation aufgetrennt. Beispielsweise erfolgt zunächst die destillative Abtrennung von Wasser und Morpholin bei Normaldruck, danach die destillative Abtrennung von weiterem Morpholin und DEG bei ca. 5 bis 20 mbar und schließlich die Isolierung von reinem DMDEE durch Rektifikation des verbleibenden Rohprodukts bei ca. 2 bis 8 mbar.

Das als Nebenprodukt anfallende Morpholin sowie evtl. unumgesetztes Diethylenglykol können wieder in die DMDEE-Synthese zurückgeführt werden.

### Beispiel

Diethylenglykol wurde zusammen mit Ammoniak und Wasserstoff über einen Vorheizer in einen bei 1,5 MPa (15 bar) Überdruck betriebenen Rohrreaktor gefahren, der mit 1,5 l (Schüttvolumen) eines Fällkatalysators der Zusammensetzung 55 Gew.% CuO und 45 Gew.-% gamma-Al₂O₃ (nach dessen letzter Wärmebehandlung und vor dessen Reduktion mit Wasserstoff) gefüllt worden war.

Die Herstellung des Katalysators erfolgte analog Beispiel 1 der DE-A-24 45 303 und vor Beginn der Umsetzung wurde der Katalysator im Wasserstoffstrom bei ca. 200 °C reduziert.

Der Reaktor hatte eine Temperatur von 215 bis 225 °C; die Kreisgasmenge betrug ca. 11 Nm³/h [Nm³ = Normkubikmeter = auf Normalbedingungen umgerechnetes Volumen]. Es wurde eine geringe Abgasmenge von ca. 300 Nl/h [Nl = Normliter = auf Normalbedingungen umgerechnetes Volumen] gefahren.

Das Molverhältnis Diethylenglykol zu Ammoniak betrug 1 : 3,6, die Katalysatorbelastung lag bei 0,4 kg Diethylenglykol und 0,23 kg Ammoniak pro 1,5 Liter Katalysator (Schüttvolumen) und Stunde.

Der Reaktoraustrag wurde in einem Abscheider entspannt und gaschromatographisch analysiert. Die Zusammensetzung war wie folgt (in GC-F1.%, ammoniak- und wasserfrei):

| | |
|---|---|
| Morpholin | 36,9 |
| N-Ethylmorpholin | 0,8 |
| Monoaminodiglykol | 1,4 |
| Diethylenglykol | 16,2 |
| 2,2'-Dimorpholinodiethylether | 40,2 |
| Sonstige | 4,5 |

Dies entsprach einem Diethylenglykol-Umsatz von 83,8 %, einer 2,2'-Dimorpholinodiethylether-Selektivität von 48 % und damit einer DMDEE-Ausbeute von 40 %.

Der Reaktoraustrag wurde anschließend destillativ aufgearbeitet. Dabei wurden zunächst in einer 1 m Kolonne bei Normaldruck Wasser, N-Ethylmorpholin und anschließend Morpholin abdestilliert. Bei ca. 10 hPa (10 mbar) wurde danach weiteres Morpholin und anschließend eine Mischung aus Monoaminodiglykol und DEG abdestilliert. Das DMDEE verblieb im Rückstand und wurde schließlich über eine 30 cm Kolonne bei ca. 5 mbar und einer Sumpftemperatur von 190 °C destilliert. Nach einer geringen Vorlaufmenge erhielt man das DMDEE mit einer Reinheit von größer 99,5 % (nach GC). Überschüssiges Ammoniak und nicht umgesetztes Diethylenglykol wurden nach der destillativen Abtrennung zurückgeführt.

## Patentansprüche

1. Verfahren zur Herstellung von 2,2'-Dimorpholinodiethylether durch Umsetzung von Diethylenglykol mit Ammoniak unter Druck und bei erhöhter Temperatur in Gegenwart von Wasserstoff und einem Hydrierkatalysator, **dadurch gekennzeichnet, dass** die katalytisch aktive Masse des Katalysators vor dessen Reduktion mit Wasserstoff
| | |
|---|---|
| 20 bis 85 Gew.-% | Aluminiumoxid (Al₂O₃), Zirkoniumdioxid (ZrO₂), Titandioxid (TiO₂) und/oder Siliziumdioxid (SiO₂), |
| | |
| 1 bis 70 Gew.-% | sauerstoffhaltige Verbindungen des Kupfers, berechnet als CuO, |
| | |
| 0 bis 50 Gew.-% | sauerstoffhaltige Verbindungen des Magnesiums, berechnet als MgO, sauerstoffhaltige Verbindungen des Chroms, berechnet als Cr₂O₃, sauerstoffhaltige Verbindungen des Zinks, berechnet als ZnO, sauerstoffhaltige Verbindungen des Bariums, berechnet als BaO, und/oder sauerstoffhaltige Verbindungen des Calciums, berechnet als CaO, und |
| | |
| weniger als 10 Gew.-% | sauerstoffhaltige Verbindungen des Nikkels, berechnet als NiO, bezogen auf die sauerstoffhaltigen Verbindungen des Kupfers, berechnet als CuO, enthält. |

2. Verfahren nach Anspruch 1, **dadurch gekennzeichnet, dass** die katalytisch aktive Masse des Katalysators vor dessen Reduktion mit Wasserstoff
| | |
|---|---|
| weniger als 5 Gew.-% | sauerstoffhaltige Verbindungen des Nikkels, berechnet als NiO, bezogen auf die sauerstoffhaltigen Verbindungen des Kupfers, berechnet als CuO, |
enthält.

3. Verfahren nach den Ansprüchen 1 bis 2, **dadurch gekennzeichnet, dass** die katalytisch aktive Masse des Katalysators vor dessen Reduktion mit Wasserstoff
| | |
|---|---|
| 30 bis 75 Gew.-% | Aluminiumoxid (Al₂O₃), Zirkoniumdioxid (ZrO₂), Titandioxid (TiO₂) und/oder Siliziumdioxid (SiO₂), |
| | |
| 5 bis 60 Gew.-% | sauerstoffhaltige Verbindungen des Kupfers, berechnet als CuO, und |
| | |
| 0 bis 20 Gew.-% | sauerstoffhaltige Verbindungen des Magnesiums, berechnet als MgO, sauerstoffhaltige Verbindungen des Chroms, berechnet als Cr₂O₃, sauerstoffhaltige Verbindungen des Zinks, berechnet als ZnO, sauerstoffhaltige Verbindungen des Bariums, berechnet als BaO, und/oder sauerstoffhaltige Verbindungen des Calciums, berechnet als CaO, |
enthält.

4. Verfahren nach den Ansprüchen 1 bis 3, **dadurch gekennzeichnet, dass** man die Umsetzung in der Gasphase durchführt.

5. Verfahren nach den Ansprüchen 1 bis 4, **dadurch gekennzeichnet, dass** man die Umsetzung bei Temperaturen von 100 bis 300°C durchführt.

6. Verfahren nach den Ansprüchen 1 bis 5, **dadurch gekennzeichnet, dass** man die Umsetzung bei Drücken von 0,1 bis 30 MPa durchführt.

7. Verfahren nach den Ansprüchen 1 bis 6, **dadurch gekennzeichnet, dass** das Molverhältnis von Ammoniak zu Diethylenglykol 1 : 1 bi 50 **:** 1 beträgt.

## Claims

1. A process for preparing bis(2-morpholinoethyl) ether by reacting diethylene glycol with ammonia under superatmospheric pressure and at elevated temperature in the presence of hydrogen and a hydrogenation catalyst, wherein the catalytically active composition of the catalyst prior to reduction with hydrogen comprises
| | |
|---|---|
| from 20 to 85% | by weight of aluminum oxide (Al₂O₃), zirconium dioxide (ZrO₂), titanium dioxide (TiO₂) and/or silicon dioxide (SiO₂), |
| | |
| from 1 to 70% | by weight of oxygen-containing compounds of copper, calculated as CuO, |
| | |
| from 0 to 50% | by weight of oxygen-containing compounds of magnesium, calculated as MgO, oxygen-containing compounds of chromium, calculated as Cr₂O₃, oxygen-containing compounds of zinc, calculated as ZnO, oxygen-containing compounds of barium, calculated as BaO, and/or oxygen-containing compounds of calcium, calculated as CaO, and |
| | |
| less than 10% | by weight of oxygen-containing compounds of nickel, calculated as NiO, based on the oxygen-containing compounds of copper, calculated as CuO. |

2. A process as claimed in claim 1, wherein the catalytically active composition of the catalyst prior to reduction with hydrogen comprises
| | |
|---|---|
| less than 5% | by weight of oxygen-containing compounds of nickel, calculated as NiO, based on the oxygen-containing compounds of copper, calculated as CuO. |

3. A process as claimed in claim 1 or 2, wherein the catalytically active composition of the catalyst prior to reduction with hydrogen comprises
| | |
|---|---|
| from 30 to 75% | by weight of aluminum oxide (Al₂O₃), zirconium dioxide (ZrO₂), titanium dioxide (TiO₂) and/or silicon dioxide (SiO₂), |
| | |
| from 5 to 60% | by weight of oxygen-containing compounds of copper, calculated as CuO, and |
| | |
| from 0 to 20% | by weight of oxygen-containing compounds of magnesium, calculated as MgO, oxygen-containing compounds of chromium, calculated as Cr₂O₃, oxygen-containing compounds of zinc, calculated as ZnO, oxygen-containing compounds of barium, calculated as BaO, and/or oxygen-containing compounds of calcium, calculated as CaO. |

4. A process as claimed in any of claims 1 to 3, wherein the reaction is carried out in the gas phase.

5. A process as claimed in any of claims 1 to 4, wherein the reaction is carried out at from 100 to 300°C.

6. A process as claimed in any of claims 1 to 5, wherein the reaction is carried out at a pressure of from 0.1 to 30 MPa.

7. A process as claimed in any of claims 1 to 6, wherein the molar ratio of ammonia to diethylene glycol is from 1:1 to 50:1.

## Revendications

1. Procédé de préparation de l'éther de 2-2'-dimorpholinodiéthylique par réaction du diéthylèneglycol avec de l'ammoniac, sous une pression et à une température élevées, en présence d'hydrogène et d'un catalyseur d'hydrogénation, **caractérisé en ce que** la masse à activité catalytique du catalyseur, avant sa réduction avec l'hydrogène, contient :
| | |
|---|---|
| 20% à 85% | en poids d'oxyde d'aluminium (Al₂O₃), de dioxyde de zirconium (ZrO₂), de dioxyde de titane (TiO₂) et/ou de dioxyde de silicium (SiO₂), |
| 1% à 70% | en poids de composés oxygénés du cuivre, exprimés en CuO, |
| 0% à 50% | en poids de composés oxygénés du magnésium, exprimés en MgO, de composés oxygénés du chrome, exprimés en Cr₂O₃, de composés oxygénés du zinc, exprimés en ZnO, de composés oxygénés du baryum, exprimé en BaO et/ou de composés oxygénés du calcium, exprimés en CaO, et |
| moins de 10% | en poids de composés oxygénés du nickel, exprimés en NiO, par rapport aux composés oxygénés du cuivre, exprimés en CuO. |

2. Procédé selon la revendication 1, **caractérisé en ce que** la masse à activité catalytique du catalyseur, avant sa réduction avec l'hydrogène, contient :
| | |
|---|---|
| moins de 5% | en poids de composés oxygénés du nickel, exprimés en NiO, par rapport aux composés oxygénés du cuivre, exprimés en CuO. |

3. Procédé selon les revendications 1 à 2, **caractérisé en ce que** la masse à activité catalytique du catalyseur, avant sa réduction avec l'hydrogène, contient :
| | |
|---|---|
| 30% à 75% | en poids d'oxyde d'aluminium (Al₂O₃), de dioxyde de zirconium (ZrO₂), de dioxyde de titane (TiO₂) et/ou de dioxyde de silicium (SiO₂), |
| 5% à 60% | en poids de composés oxygénés du cuivre, exprimés en CuO, |
| 0% à 20% | en poids de composés oxygénés du magnésium, exprimés en MgO, de composés oxygénés du chrome, exprimés en Cr₂O₃, de composés oxygénés du zinc, exprimés en ZnO, de composés oxygénés du baryum, exprimés en BaO et/ou de composés oxygénés du calcium, exprimés en CaO. |

4. Procédé selon les revendications 1 à 3, **caractérisé en ce que** l'on réalise la réaction en phase gazeuse.

5. Procédé selon les revendications 1 à 4, **caractérisé en ce que** l'on réalise la réaction à des températures allant de 100°C à 300°C.

6. Procédé selon les revendications 1 à 5, **caractérisé en ce que** l'on réalise la réaction sous des pressions allant de 0,1 à 30 MPa.

7. Procédé selon les revendications 1 à 6, **caractérisé en ce que** le rapport molaire d'ammoniac au diéthylèneglycol est compris entre 1:1 et 50:1.
